(19) Europäisches Patentamt
European Patent Office
Office européen des brevets

(11) Veröffentlichungsnummer: **0 687 669 A1**

# (12) EUROPÄISCHE PATENTANMELDUNG

(21) Anmeldenummer: **95101867.0**

(22) Anmeldetag: **11.02.95**

(51) Int. Cl.6: **C07C 217/84**, C07C 211/52, A61K 7/13

(30) Priorität: **18.06.94 DE 4421397**

(43) Veröffentlichungstag der Anmeldung: **20.12.95 Patentblatt 95/51**

(84) Benannte Vertragsstaaten: **DE ES FR GB IT**

(71) Anmelder: **Wella Aktiengesellschaft**
**Berliner Allee 65**
**D-64274 Darmstadt (DE)**

(72) Erfinder: **Balzer, Wolfgang R., Dr.**
**Schlesier Strasse 9a**
**E-64665 Alsbach (DE)**
Erfinder: **Frank, Anke**
**Alte Falterstrasse 29**
**D-65933 Frankturt (DE)**
Erfinder: **Weinges, Alexa, Dr.**
**Langgewann 9**
**D-69121 Heidelberg (DE)**

## (54) Mittel zu oxidativen Färbung von Haaren und neue 2-Alkylamino-4-amino-1-alkylbenzole

(57) Gegenstand der vorliegenden Erfindung ist ein Mittel zur oxidativen Färbung von Haaren mit einem Gehalt an mindestens einer Kupplersubstanz der allgemeinen Formel

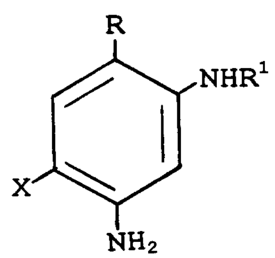

(I),

wobei R einen geradkettigen oder verzweigten $C_1$- bis $C_4$-Alkylrest, X Chlor oder einen geradkettigen oder verzweigten $C_1$- bis $C_4$-Alkoxyrest und $R^1$ einen geradkettigen oder verzweigten $C_1$- bis $C_4$-Alkylrest oder einen geradkettigen oder verzeigten $C_2$- bis $C_4$- Hydroxyalkylrest darstellt sowie neue 2-Alkylamino-4-amino-1-alkylbenzole.

Die Kupplersubstanzen der Formel (I) sind durch einfache chemische Reaktionen zugänglich, gut wasserlöslich und weisen, insbesondere als Bestandteil der hier beschriebenen Haarfärbemittel, eine guter Lagerstabilität auf.

Die mit dem erfindungsgemäßen Haarfärbemittel erzielbaren Farbergebnisse zeichnen sich, neben der Vielfalt der Farbnuancen, insbesondere durch die Farbintensität der Rottöne und eine gute Farbreinheit der Blautöne aus.

EP 0 687 669 A1

Gegenstand der Erfindung ist ein Mittel zur oxidativen Färbung von Haaren auf der Basis von in 5-Stellung substituierten 2-Alkylamino-4-amino-1-alkylbenzolen als Kupplersubstanzen sowie neue in 5-Stellung substituierte 2-Alkylamino-4-amino-1-alkylbenzole.

In der Haarfärbepraxis haben Oxidationsfarbstoffe eine wesentliche Bedeutung erlangt. Hierbei werden die Farbstoffe durch oxidative Kupplung von Entwicklersubstanzen und Kupplersubstanzen im Haarschaft erzeugt. Dies führt zu sehr intensiven Haarfärbungen mit sehr guter Farbechtheit. Außerdem können durch die Kombination geeigneter Entwickler- und Kupplersubstanzen unterschiedliche Farbnuancen erzeugt werden.

Als Entwicklersubstanzen werden bevorzugt 2,5-Diaminotoluol, 1,4-Diaminobenzol, 2-(2'-Hydroxyethyl)-1,4-diaminobenzol, 4-Aminophenol, 4-Amino-2-aminomethylphenol und 4-Amino-3-methylphenol sowie substituierte 4,5-Diaminopyrazole verwendet.

Die bevorzugt verwendeten Kupplersubstanzen sind dabei m-Phenylendiamin und dessen Derivate, wie zum Beispiel 2,4-Diamino-phenoxyethanol, 2-Amino-4-(2'-hydroxyethyl)- aminoanisol oder Pyridinderivate wie 3,5-Diamino-2,6-dimethoxypyridin als Blaukuppler, 1-Naphthol, m-Aminophenol und dessen Derivate, wie 2-Amino-4-chlor-6-methylphenol, 5-Amino-2-methylphenol, 4-Amino-2-hydroxyphenoxyethanol, 4-Amino-5-fluor-2-hydroxytoluol und 4-Amino-5-ethoxy-2-hydroxytoluol als Rotkuppler sowie Resorcin, 4-Chlorresorcin, 4,6-Dichlorresorcin, 2-Methylresorcin, 4-Hydroxy-1,2-methylendioxybenzol, 4-(2'-Hydroxyethyl)amino-1,2-methylendioxybenzol und 4-Hydroxyindol als Kuppler für den Braun-Blond-Bereich.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwickler- und Kupplerkomponenten eine breite Palette verschiedener Farbnuancen erzeugt werden kann.

Die zur Zeit in Haarfärbemitteln zur Erzeugung insbesondere von roten und klaren blauen Farbtönen verwendeten Kupplersubstanzen können die vorstehend genannten Anforderungen jedoch nicht zufriedenstellend erfüllen. Ebenso sind diese Substanzen in ihren dermatologischen Eigenschaften für manche Anwender nicht optimal. Es besteht daher das Bedürfnis, weitere Kupplersubstanzen mit verbesserten dermatologischen und toxikologischen Eigenschaften zu finden. Außerdem weisen diese Verbindungen Mängel in ihrer Stabilität auf, was sich insbesondere durch ein vorzeitiges Ausbleichen unter Lichteinfluß zeigt.

Die aus der DE-OS 42 06 416 bekannten 5-Halogen-2,4- bis -(alkylamino)-1-alkylbenzole zeigen zwar im Amestest keine mutagene Wirkung, jedoch sind die Intensitäten der mit dieser Kupplersubstanz erzielten Ausfärbungen aufgrund ihrer guten Löslichkeit nicht immer zufriedenstellend.

Die in der DE-OS 36 22 784 beschriebenen Kupplersubstanzen 2,4-Diamino-5-ethoxytoluol beziehungsweise 2,4-Diamino-5-(2'hydroxyethyl)oxytoluol besitzen zwar gute toxikologische Eigenschaften, zeigen jedoch mit Entwicklern wie p-Aminophenol oder dessen Derivaten nur sehr farbschwache Anfärbungen.

Das aus der DE-OS 40 28 661 bekannte 5-Fluor-2,4-diamino-toluol zeigt zwar im Amestest keine mutagene Wirkung, jedoch läßt sich ein Defizit in der Lichtstabilität der mit dieser Kupplersubstanz erzielten Ausfärbungen feststellen.

Demgemäß bestand die Aufgabe, ein Haarfärbemittel zur oxidativen Färbung von Haaren auf der Basis von in der Haarfärbung üblichen Entwicklersubstanzen mit einem Gehalt an neuen Kupplersubstanzen zur Verfügung zu stellen, bei dem die zuvor genannten Anforderungen an die anwendungstechnischen Eigenschaften der neuen Kupplersubstanz erfüllt werden.

Hierzu wurde nun gefunden, daß ein Mittel zur oxidativen Färbung von Haaren auf der Basis einer Entwicklersubstanz-Kupplersubstanz-Kombination sowie gegebenenfalls anderer Farbkomponenten und in Haarfärbemitteln üblichen Zusätzen, welches mindestens eine Kupplersubstanz der allgemeinen Formel

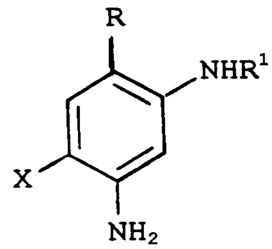

(I),

wobei R einen gradkettigen oder verzweigten $C_1$-bis $C_4$-Alkylrest, X Chlor oder einen geradkettigen oder verzweigten $C_1$- bis $C_4$-Alkoxyrest und $R^1$ einen geradkettigen oder verzweigten $C_1$- bis $C_4$-Alkylrest oder einen geradkettigen oder verzweigten $C_2$- bis $C_4$-Hydroxyalkylrest darstellt, oder deren physiologisch verträgliches wasserlösliches Salz enthält, die gestellte Aufgabe in hervorragendem Maß löst.

Im Gegensatz zu den erfindungsgemäßen Verbindungen der Formel (I) weisen die isomeren Verbindungen der Formel (II)

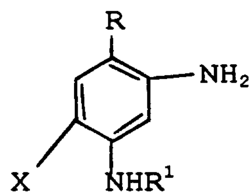

(II),

wobei R,X und $R^1$ die vorstehend angegebene Bedeutung haben, ebenso wie die in 5-Stellung nicht substituierten Verbindungen eine nicht ausreichende Beständigkeit der Ausfärbungen auf.

Die Kupplersubstanzen der allgemeinen Formel (I) sind durch einfache chemische Reaktionen aus technisch zugänglichen Ausgangsverbindungen erhältlich und ergeben mit Entwicklersubstanzen wie p-Phenylendiamin klare blaue Ausfärbungen ohne Rotstich. Mit Entwicklersubstanzen vom Typ p-Aminophenol lassen sich farbsatte, rote Färbungen erzielen, deren Farbintensität sogar größer ist als bei Ausfärbungen mit den unsubstituierten 2-Alkylamino-4-amino-1-alkylbenzolen.

Die Kupplersubstanzen der allgemeinen Formel (I) sind gut in Wasser löslich und weisen, insbesondere als Bestandteil der hier beschriebenen Haarfärbemittel, eine ausgezeichnete Lagerstabilität auf.

In dem hier beschriebenen Haarfärbemittel sollen die erfindungsgemäßen Kupplersubstanzen der Formel (I), in einer für die Haarfärbung ausreichenden Menge, vorzugsweise in einer Menge von 0,01 bis 5 Gewichtsprozent, besonders bevorzugt 0,1 bis 3 Gewichtsprozent, enthalten sein.

Von den Kupplersubstanzen der allgemeinen Formel (I) sind in dem Haarfärbemittel bevorzugt 4-Amino-2-(2'-hydroxyethyl)amino-5-ethoxy-toluol, 4-Amino-2-(2'-hydroxyethyl)amino-5-methoxy-toluol und 4-Amino-2-(2'-hydroxyethyl)amino-5-chlor-toluol enthalten.

Außerdem können in dem Haarfärbemittel zusätzlich 0,01 bis 5 Gewichtsprozent, bevorzugt 0,1 bis 3 Gewichtsprozent, mindestens einer weiteren Kupplersubstanz, beispielsweise Resorcin, 4-Chlorresorcin, 4,6-Dichlorresorcin, 2-Methylresorcin, 2-Amino-4-(2'-hydroxyethylamino)anisol, 4-Amino-5-fluor-2-hydroxytoluol, 4-Amino-5-ethoxy-2-hydroxytoluol, m-Phenylendiamin, 5-Amino-2-methylphenol, 2,4-Diamino-phenoxyethanol, 1-Naphthol, m-Aminophenol, 3-Amino-4-chlor-6-methylphenol, 3-Amino-2-methylphenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol und 3,5-Diamino-2,6-dimethoxypyridin, enthalten sein.

Von den Entwicklersubstanzen kommen als Bestandteil der erfindungsgemäßen Haarfärbemittel vor allem 1,4-Diaminobenzol, 2,5-Diaminotoluol, 2-(2'-Hydroxyethyl)-1,4-diaminobenzol, 4-Aminophenol, 4-Amino-2-aminomethylphenol, 4-Amino-3-methylphenol, 4-Amino-2-methoxymethylphenol, 4-Amino-2-ethoxymethylphenol, 4,5-Diamino-1-methyl-pyrazol, 4,5-Diamino-1-isopropyl-pyrazol, 1-Benzyl-4,5-diamino-pyrazol, 4,5-Diamino-1-methylbenzyl-pyrazol sowie Tetraaminopyrimidin oder deren physiologisch verträgliche Salze in Betracht. Die in dem erfindungsgemäßen Mittel enthaltene Menge der Entwicklersubstanzen soll vorzugsweise 0,01 bis 5 Gewichtsprozent, besonders bevorzugt jedoch 0,1 bis 3,0 Gewichtsprozent, betragen.

Die üblichen Kuppler- und Entwicklersubstanzen können in dem erfindungsgemäßen Haarfärbemittel jeweils einzeln oder im Gemisch miteinander enthalten sein. Die Gesamtmenge der in dem hier beschriebenen Haarfärbemittel enthaltenen Entwicklersubstanz-Kupplersubstanz-Kombination soll etwa 0,1 bis 5,0 Gewichtsprozent, vorzugsweise 0,5 bis 4,0 Gewichtsprozent, betragen.

Die Entwicklersubstanzen werden im allgemeinen in etwa äquimolaren Mengen, bezogen auf die Kupplersubstanzen, eingesetzt. Es ist jedoch nicht nachteilig, wenn die Entwicklersubstanzen diesbezüglich in einem gewissen Überschuß oder Unterschuß vorhanden sind.

Weiterhin kann das erfindungsgemäße Haarfärbemittel zusätzlich auch andere mit sich selbst kuppelnde beziehungsweise direktziehende Farbkomponenten, beispielsweise 6-Amino-2-methylphenol und 2-Amino-5-methylphenol sowie ferner übliche direktziehende Farbstoffe, zum Beispiel Triphenylmethanfarbstoffe wie C. I. Basic Violet 14 (C. I. 42 510) und C. I. Basic Violet 2 (C. I. 42 520), aromatische Nitrofarbstoffe wie 2-Amino-4,6-dinitrophenol, 2-Amino-5-(2'-hydroxyethylamino)nitrobenzol und 4-(2'-Hydroxyethylamino)-3-nitrotoluol, 1-(2'-Ureidoethyl) amino-4-nitrobenzol und Azofarbstoffe wie C. I. Acid Brown 4 (C. I. 14 805) sowie Dispersionsfarbstoffe, wie beispielsweise 1,4-Diaminoanthrachinon und 1,4,5,8-Tetraaminoanthrachinon in einer Menge von etwa 0,1 bis 4 Gewichtsprozent enthalten.

Weitere geeignete direkt auf das Haar aufziehende Farbstoffe sind beispielsweise im Buch von J. C. Johnson „Hair Dyes", Noyes Data Corp., Park Ridge, USA (1973), Seiten 3 - 91 und 113 - 139 (ISBN: 0-8155-0477-2) beschrieben.

Selbstverständlich können die Kuppler- und Entwicklersubstanzen sowie die anderen Farbkomponenten, sofern es Basen sind, auch in Form der physiologisch verträglichen Säureadditionssalze, wie beispielsweise als Hydrochlorid beziehungsweise Sulfat oder - sofern sie aromatische OH-Gruppen besitzen - in Form der Salze mit Basen, zum Beispiel als Alkaliphenolate, eingesetzt werden.

Darüberhinaus können in dem Haarfärbemittel noch übliche kosmetische Zusätze, beispielsweise Antioxidantien wie Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrig-alkoholische Lösung, sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphotheren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxyethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert zwischen 6,0 und 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 3 bis 12-prozentigen, vorzugsweise 6-prozentigen, wäßrigen Lösung in Betracht. Ebenso ist es möglich, die Färbung unter Einwirkung von Luftsauerstoff, das heißt ohne Zusatz eines Oxidationsmittels, zu entwickeln. Wird eine 6-prozentige Wasserstoffperoxidlösung als Oxidationsmittel verwendet,so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet.

Man läßt das Gemisch bei 15 bis 50° C etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen

Säure, wie zum Beispiel Zitronensäure oder Weinsäure, nachgespült. Anschließend wird das Haar getrocknet.

Das erfindungsgemäße Haarfärbemittel führt zu Haarfärbungen mit ausgezeichneten Echtheitseigenschaften, insbesondere was die Licht-, Wasch- und Reibechtheit anbetrifft, und läßt sich mit Reduktionsmitteln wieder abziehen. Hinsichtlich der färberischen Möglichkeiten bietet das erfindungsgemäße Haarfärbemittel je nach Art und Zusammensetzung der Farbkomponenten eine breite Palette verschiedener Farbnuancen. Bemerkenswert ist die große Farbintensität der erzielbaren roten und die Farbreinheit der erzielbaren blauen Färbungen. Schließlich ist mit Hilfe des beschriebenen Haarfärbemittels auch eine Anfärbung von ergrautem und chemisch nicht vorgeschädigtem Haar problemlos und mit sehr guter Deckkraft möglich. Die dabei erhaltenen Färbungen sind, unabhängig von der unterschiedlichen Struktur des Haares, gleichmäßig und sehr gut reproduzierbar.

Die in 5-Stellung substituierten 2-Alkylamino-4-amino-1-alkylbenzole der Formel (I) lassen sich einfach aus den technisch verfügbaren 2-Amino-1-alkyl-benzolen (III) herstellen. Hierzu werden zunächst durch Nitrierung in 4-Stellung die entsprechenden 2-Amino-5-halogen-4-nitro-1-alkylbzenzole (IV) hergestellt, wobei R und X die vorstehend angegebene Bedeutung haben:

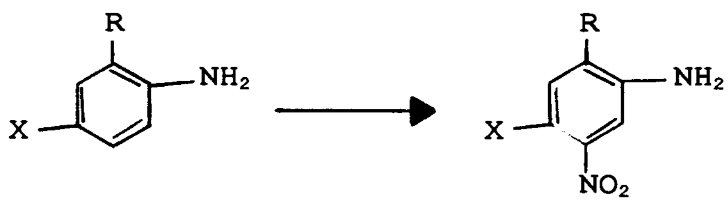

(III) (IV)

Für die Nitrierung kommen die in der organischen Synthese üblichen Nitrierungsreagenzien in Frage, wobei ein Gemisch aus Schwefelsäure und Salpetersäure bevorzugt ist.

Ausgehend vom 2-Amino-5-halogen-4-nitro-1-alkylbenzol (IV) sind die neuen Kupplersubstanzen der allgemeinen Formel (I) auf zwei Wegen zugänglich.

1. Das 2-Amino-5-halogen-4-nitro-1-alkylbenzol (IV) wird mit dem entsprechenden $C_2$- bis $C_4$-Chlorameisensäure-chloralkylester zu den hydroxyalkylierten Verbindungen der allgemeinen Formel (V), wobei $R^2$ einen geradkettigen oder verzweigten $C_2$- bis $C_4$-Hydroxyalkylrest darstellt und R und X die vorstehend angegebene Bedeutung haben, umgesetzt. Die Reduktion der Nitrogruppe wird mit für diesen Syntheseschritt bekannten Reagenzien, beispielsweise Wasserstoff in Gegenwart eines geeigneten Katalysators, beispielsweise Palladium/Aktivkohle, durchgeführt und führt zu den hydroxyalkylierten erfindungsgemäßen Verbindungen der allgemeinen Formel (Ia)

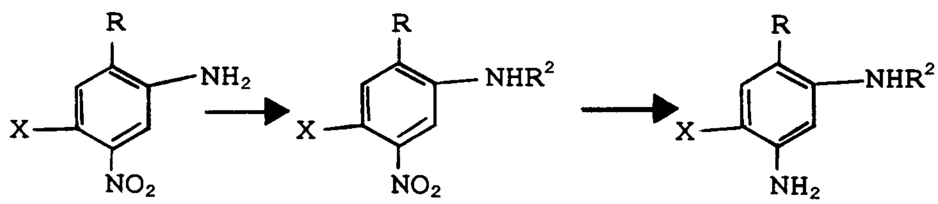

(IV) (V) (I a)

2. Die erfindungsgemäßen Verbindungen der Formel (Ib), bei denen die Aminogruppe mit einem geradkettigen oder verzweigten $C_1$- bis $C_4$-Alkylrest substituiert sind, lassen sich aus den entsprechenden 2-Amino-5-halogen-4-nitro-1-alkylbenzolen (IV) in vier Syntheseschritten gemäß dem nachfolgend angegebenen Schema durch Umsetzung mit einem für die Aminogruppe üblichen Schutzgruppenreagenz, beispielsweise Benzolsulfonychlorid, Alkylierung mit einem üblichen Alkylierungsreagenz, beispielsweise $C_1$- bis $C_4$-Alkyljodiden und anschließende Entfernung der Schutzgruppen sowie Reduktion der Nitrogruppe, wobei $R^3$ einen geradkettigen oder verzweigten $C_1$- bis $C_4$-Alkylrest und Y eine für Aminogruppen übliche Schutzgruppe darstellt und R und X die vorstehend angegebene Bedeutung haben, herstellen.

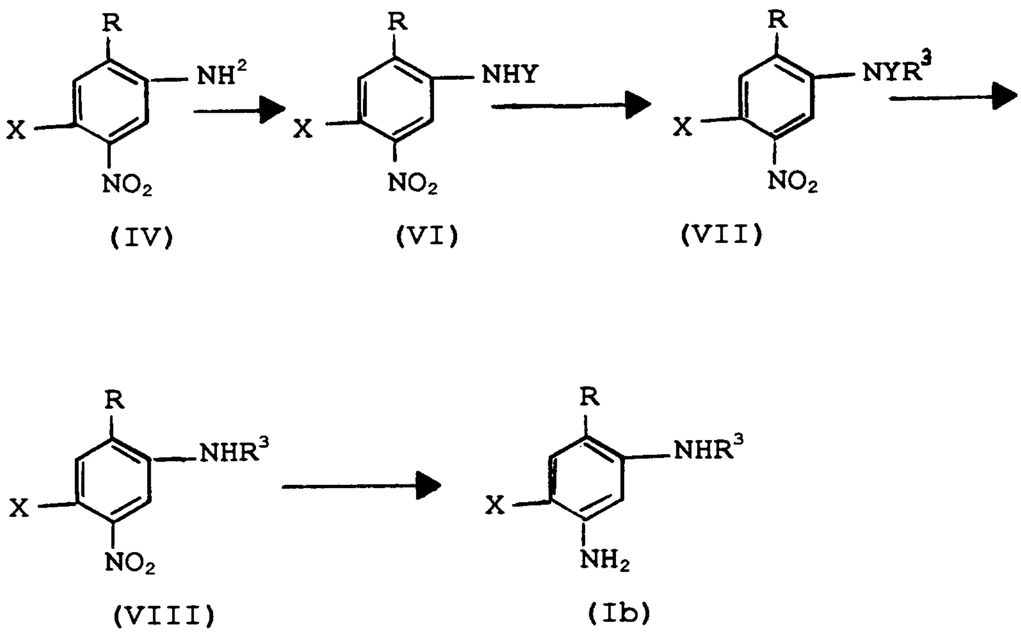

(IV) (VI) (VII)

(VIII) (Ib)

Die nachfolgenden Beispiele sollen den Gegenstand der Erfindung näher erläutern, ohne den Erfindungsgegenstand auf diese Beispiele zu beschränken.

**Beispiele** für Haarfärbemittel

Beispiel 1: Haarfärbemittel in Gelform

0,08 g 4-Amino-2-(2'-hydroxyethyl)amino-5-methoxy-toluol
1,00 g 2-(2'-Hydroxyethyl)-1,4-diaminobenzolsulfat
0,35 g Resorcin
0,05 g m-Aminophenol
0,40 g Natriumsulfit, wasserfrei
15,00 g Ölsäure
7,00 g Isopropanol
10,00 g Ammoniak (22-prozentige wäßrige Lösung)
66,12 g Wasser
100,00 g

Man vermischt kurz vor der Anwendung 50 g dieses Haarfärbemittels in Gelform mit 50 ml einer 6-prozentigen Wasserstoffperoxidlösung. Das Gemisch wird sodann auf blonde menschliche Haare aufgetragen. Nach einer Einwirkungszeit von dreißig Minuten bei 40 Grad Celsius wird das Haar mit Wasser gespült und sodann getrocknet. Das Haar hat eine natürliche braune Färbung angenommen.

Beispiel 2: Haarfärbelösung

0,22 g 4-Amino-2-(2'-hydroxyethyl)amino-5-methoxytoluol
1,26 g p-Aminophenol
0,12 g Resorcin
0,10 g m-Aminophenol
1,05 g 5-Amino-2-methyl-phenol
0,40 g Natriumsulfit, wasserfrei
10,00 g Laurylalkohol-diglykolethersulfat (28-prozentige wäßrige Lösung)
10,00 g Isopropanol
20,00 g Ammoniak (22-prozentige wäßrige Lösung)
56,85 Wasser, vollentsalzt
100,00 g

50 g der vorstehenden Haarfärbelösung werden kurz vor der Anwendung mit 50 ml einer 6-prozentigen

Wasserstoffperoxidlösung vermischt. Das Gemisch wird sodann auf blonde menschliche Haare aufgetragen. Nach einer Einwirkungszeit von dreißig Minuten bei vierzig Grad Celsius wird das Haar mit Wasser gespült und getrocknet. Das Haar ist in einem modischen Bordeauxrot gefärbt.

## Vergleichsbeispiele

Zum Vergleich der Farbstabilitäten wurden als Farbstoffe jeweils eine der erfindungsgemäßen Kupplersubstanzen der allgemeinen Formel (I)
4-Amino-2-(2'-hydroxyethyl)amino-5-methoxy-toluol
4-Amino-2-(2'-hydroxyethyl)amino-5-chlor-toluol
oder die bekannten Kupplersubstanzen
2,4-Diamino-5-ethoxy-toluol
2,4-Diamino-5-fluor-toluol
in Kombination mit einer äquimolaren Menge einer der bekannten Entwicklersubstanzen
1,4-Diamino-benzol (Beispiele I, IV, VII und X)
2,5-Diamino-toluol (Beispiele II, V, VIII und XI)
2-(2'Hydroxyethyl)-1,4-diamino-benzol (Beispiele III, VI, IX und XII)
in einer Haarfärbelösung eingesetzt.

Beispiel I: Färbelösung

10,0000 g eines Gemisches aus den Kaliumsalzen der Kokosfettsäure und der Ölsäure
10,0000 g Isopropanol
2,4531 g 4-Amino-2-(2'-hydroxyethyl)amino-5-methoxy-toluol
1,3518 g 1,4-Diamino-benzol
10,0000 g Ammoniak (25 prozentige wäßrige Lösung)
66,1951 g Wasser
100,0000 g

Beispiel II. Färbelösung

10,0000 g eines Gemisches aus den Kaliumsalzen der Kokosfesttsäure und der Ölsäure
10,0000 g Isopropanol
2,4531 g 4-Amino-2-(2'-hydroxyethyl)amino-5-metoxy-toluol
1,5271 g 1,4-Diamino-toluol
10,0000 g Ammoniak (25-prozentige wäßrige Lösung)
66,0198 g Wasser
100,0000 g

Beispiel III: Färbelösung

10,0000 g eines Gemisches aus den Kaliumsalzen der Kokosfettsäure und der Ölsäure
10,0000 g Isopropanol
2,4531 g 4-Amino-2-(2'-hydroxyethyl)amino-5-methoxy-toluol
1,9025 g 2-(2'Hydroxyethyl)-1,4-diamino-benzol
10,0000 g Ammoniak (25-prozentige wäßrige Lösung)
65,6444 g Wasser
100,0000 g

Beispiel IV: Färbelösung

10,0000 g eines Gemisches aus den Kaliumsalzen der Kokosfettsäure und der Ölsäure
10,0000 g Isopropanol
2,5084 g 4-Amino-2-(2'-hydroxyethyl)amino-5-chlor-toluol
1,3518 g 1,4-Diamino-benzol
10,0000 g Ammoniak (25-prozentige wäßrige Lösung)
66,1398 g Wasser

100,0000 g

Beispiel V: Färbelösung

10,0000 g eines Gemisches aus den Kaliumsalzen der Kokosfettsäure und der Ölsäure
10,0000 g Isopropanol
2,5084 g 4-Amino-2-(2'-hydroxyethyl)amino-5-chlor-toluol
1,5271 g 2,5-Diamino-toluol
10,0000 g Ammoniak (25-prozentige wäßrige Lösung)
65,9645 g Wasser
100,000 g

Beispiel VI: Färbelösung

10,0000 g eines Gemisches aus den Kaliumsalzen der Kokosfettsäure und der Ölsäure
10,0000 g Isopropanol
2,5084 g 4-Amino-2-(2'-hydroxyethyl)amino-5-chlor-toluol
1,9025 g 2-(2'-hydroxyethyl)-1,4-diamino-benzol
10,0000 g Ammoniak (25-prozentige wäßrige Lösung)
65,5891 g Wasser
100,0000 g

Beispiel VII: Färbelösung nach dem Stand der Technik

10,0000 g eines Gemisches aus den Kaliumsalzen der Kokosfettsäure und der Ölsäure
10,0000 g Isopropanol
2,0778 g 2,4-Diamino-5-ethoxy-toluol
1,3518 g 1,4-Diamino-benzol
10,0000 g Ammoniak (25-prozentige wäßrige Lösung)
66,5704 g Wasser
100,0000 g

Beispiel VIII: Färbelösung nach dem Stand der Technik

10,0000 g eines Gemisches aus den Kaliumsalzen der Kokofettsäure und der Ölsäure
10,0000 g Isopropanol
2,0778 g 2,4-Diamino-5-ethoxy-toluol
1,5271 g 1,5-Diamino-toluol
10,0000 g Ammoniak (25-prozentige wäßrige Lösung)
66,3951 g Wasser
100,0000 g

Beispiel IX: Färbelösung nach dem Stand der Technik

10,0000 g eines Gemisches aus den Kaliumsalzen der Kokosfettsäure und der Ölsäure
10,0000 g Isopropanol
2,0778 g 2,4-Diamino-5-ethoxy-toluol
1,9025 g 2-(2'-Hydroxyethyl)-1,4-diamino-benzol
10,0000 g Ammoniak (25-prozentige wäßrige Lösung)
66,0197 g Wasser
100,0000 g

Beispiel X: Färbelösung nach dem Stand der Technik

10,0000 g eines Gemisches aus den Kaliumsalzen der Kokosfettsäure und der Ölsäure
10,0000 g Isopropanol
1,7520 g 2,4-Diamino-5-fluor-toluol
1,3518 g 1,4-Diamino-benzol

10,0000 g Ammoniak (25-prozentige wäßrige Lösung)
66,8962 g Wasser
100,0000 g

Beispiel XI: Färbelösung nach dem Stand der Technik

10,0000 g eines Gemisches aus den Kaliumsalzen der Kokosfettsäure und der Ölsäure
10,0000 g Ölsäure
1,7520 g 2,4-Diamino-5-fluor-toluol
1,5271 g 2,5-Diamino-toluol
10,0000 g Ammoniak (25-prozentige wäßrige Lösung)
66,7209 g Wasser
100,0000 g

Beispiel XII: Färbelösung nach dem Stand der Technik

10,0000 g eines Gemisches aus den Kaliumsalzen der Kokosfettsäure und der Ölsäure
10,0000 g Isopropanol
1,7520 g 2,4-Diamino-5-fluor-toluol
1,9025 g 2-(2'-Hydroxyethyl)-1,4-diamino-benzol
10,0000 g Ammoniak (25-prozentige wäßrige Lösung)
66,3455 g Wasser
100,0000 g

Jeweils 5 g der Färbelösungen der Beispiele I bis XII wurden kurz vor der Anwendung jeweils mit 5 g einer 6-prozentigen Wasserstoffperoxidlösung vermischt. Das Gemisch wurde sodann jeweils auf weiße Büffelhaarsträhnchen aufgetragen. Nach einer Einwirkungszeit von 30 Minuten bei 40 Grad Celsius wurden die Büffelhaarsträhnchen mit Wasser gespült und getrocknet.

Schließlich wurden die Farbmeßwerte der gefärbten Büffelhaarsträhnchen mit einem Farbmeßgerät (Chromameter CR 200 der Firma Minolta) bestimmt. Hierbei bedeutet Y die Helligkeit gegenüber einer rein weißen Oberfläche (Y = 100 %), das heißt je größer der Y-Wert ist, umso heller ist die Färbung.

Die Parameter x, y und z bedeuten den roten, grünen und blauen Anteil des jeweiligen Farbtons, wobei x und y gemessen werden und z sich aus der Formel $z = 1-(x+y)$ errechnen läßt.

Die Farbmeßwerte der gefärbten Büffelhaarsträhnchen sind in Tabelle 1 angegeben (die Werte in Klammern wurden nach 3 Wochen gemessen):

Tabelle 1: Farbmeßwerte

| Färbelösung | Y | x | y | z |
|---|---|---|---|---|
| Beispiel I | 2,4(2,6) | 0,239(0,245) | 0,198(0,210) | 0,563(0,545) |
| Beispiel II | 2,8(2,8) | 0,227(0,237) | 0,192(0,208) | 0,581(0,555) |
| Beispiel III | 3,2(3,2) | 0,223(0,231) | 0,187(0,200) | 0,584(0,569) |
| Beispiel IV | 2,3(2,5) | 0,256(0,266) | 0,226(0,247) | 0,518(0,487) |
| Beispiel V | 2,9(2,9) | 0,234(0,254) | 0,201(0,233) | 0,565(0,513) |
| Beispiel VI | 2,8(2,8) | 0,228(0,249) | 0,190(0,224) | 0,582(0,527) |
| Beispiel VII | 2,5(2,7) | 0,244(0,261) | 0,209(0,237) | 0,547(0,502) |
| Beispiel VIII | 3,2(3,2) | 0,224(0,247) | 0,191(0,225) | 0,585(0,528) |
| Beispiel IX | 3,8(3,8) | 0,223(0,245) | 0,192(0,221) | 0,595(0,534) |
| Beispiel X | 2,2(2,4) | 0,240(0,267) | 0,197(0,244) | 0,563(0,489) |
| Beispiel XI | 2,5(2,5) | 0,223(0,266) | 0,181(0,248) | 0,536(0,486) |
| Beispiel XII | 3,0(3,0) | 0,219(0,250) | 0,177(0,227) | 0,604(0,523) |

Im folgenden soll gezeigt werden, daß das erfindungsgemäße 4-Amino-2-(2'-hydroxyethyl)-amino-4-chlor-toluol (A) auch gegenüber dem Stellungsisomeren 2-Amino-4-(2'-hydroxyethyl)amino-5-chlor-toluol (B) bessere Färbeeigenschaften aufweist.

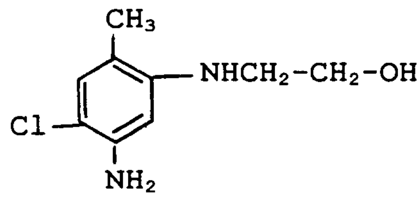

(A)

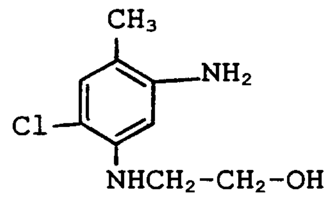

(B)

Hierzu wurden die mit einer Haarfärbelösung gemäß Beispiel IV, V und VI erhaltenen Ausfärbungen mit Ausfärbungen verglichen, bei denen in der entsprechenden Haarfärbelösung das erfindungsgemäße 4-Amino-2-(2'-hydroxyethyl)amino-4-chlor-toluol durch das isomere 2-Amino-4-(2'-hydroxyethyl)amino-5-chlor-toluol mengengleich ersetzt wurde (Beispiele IVa, Va und VIa). Die Bestimmung der Farbmeßwerte erfolgte in der vorstehend beschriebenen Weise, wobei die erste Messung unmittelbar nach der Haarfärbebehandlung erfolgte, während die zweite Messung 3 Wochen nach der Haarfärbebehandlung durchgeführt wurde.

Die Farbmeßwerte sind in der nachfolgenden Tabelle 2 zusammengefaßt. Die Werte in Klammern wurden nach 3 Wochen bestimmt.

Tabelle 2: Farbmeßwerte

| | Y | x | y | z |
|---|---|---|---|---|
| Beispiel IV | 2,3(2,5) | 0,256(0,266) | 0,226(0,247) | 0,518(0,487) |
| Beispiel V | 2,9(2,9) | 0,234(0,254) | 0,201(0,233) | 0,565(0,513) |
| Beispiel VI | 2,8(2,8) | 0,228(0,249) | 0,190(0,224) | 0,582(0,527) |
| Beispiel IVa | 2,7(2,7) | 0,245(0,288) | 0,208(0,240) | 0,547(0,472) |
| Beispiel Va | 3,1(3,4) | 0,231(0,292) | 0,192(0,235) | 0,577(0,473) |
| Beispiel VIa | 4,2(4,2) | 0,227(0,284) | 0,190(0,225) | 0,583(0,491) |

## Herstellungsbeispiele

Beispiel A: Herstellung von 4-Amino-2-(2'-hydroxyethyl)amino-5-methoxytoluol

Stufe 1: 2-Amino-5-fluor-4-nitro-toluol

1,3 g (10 mmol) 4-Fluor-2-methyl-anilin werden in 10 ml konzentrierter $H_2SO_4$ vorgelegt und auf -10 °C abgekühlt. Eine kalte Lösung von 0,4 ml (10 mmol) rauchender $HNO_3$ in 2 ml konzentrierter $H_2SO_4$ wird langsam zugetropft.

Die Mischung wird 3 Stunden bei 0 °C gerührt und anschließend auf Eis gegossen. Das ausgefallene Produkt wird abgesaugt und nachgewaschen. Man erhält 0,85 g (50 % der Theorie) gelbgefärbte Kristalle

mit einem Schmelzpunkt von 178 °C.

$^1$H-NMR ($D_6$-DMSO): δ = 2,24 (s; 3H,-$CH_3$) 5,78 (br.s; 2H,-$NH_2$ tauscht mit $D_2O$ aus) 7,28 (d,J = 12Hz; 1H, 6-H) 7,57 (d,J = 6,8 Hz; 1H, 3-H)

MS (70eV): m/e = 170 ($M^{+\cdot}$)

Stufe 2: 2-Chlorethyl-N-[(4-fluor-2-methyl-5-nitro)phenyl]-carbamat

5 g (29 mmol) 2-Amino-5-fluor-4-nitro-toluol werden mit 2,5 g Kalziumcarbonat in 200 ml Dioxan auf 70 °C erwärmt, mit 3,1 ml Chlorameisensäurechlorethylester versetzt und 3 Stunden bei 100 °C erhitzt. Die Lösung wird heiß filtriert und das Filtrat auf 500 ml Eiswasser gegeben. Der ockerfarbene Niederschlag wird abgesaugt und aus einem Ethanol-Wasser-Gemisch umkristallisiert. Es werden 6,6 g (81% der Theorie) 2-Chlorethyl-N-[(4-fluor-2-methyl-5-nitro)-phenyl]-carbamat mit einem Schmelzpunkt von 113 °C erhalten.

$^1$H-NMR ($D_6$-DMSO): δ = 2,33 (s; 3H,-$CH_3$) 3,88 (t; 2H, $C\underline{H_2}Cl$) 4,38 (t, 2H, $C\underline{H_2}CH_2Cl$) 7,49 (d,J = 12,3 Hz; 1H, 6-H) 8,22 (d,J = 7,3 Hz; 1H, 3-H) 9,44 (br. s; 2H, -NH, tauscht mit $D_2O$ aus)

MS (70 ev): m/e = 276 ($M^{+\cdot}$)

Stufe 3: 2-(2'-Hydroxyethyl)amino-4-nitro-5-methoxy-toluol

10 g (36 mmol)-2-Chlorethyl-N-[4-fluor-2-methyl-5-nitro)phenyl]-carbamat werden mit 120 ml einer Mischung aus 10-prozentiger NaOH und Methanol im Verhältnis 2:1 versetzt und 1 Stunde unter Rückfluß erhitzt. Nach dem Abkühlen wird die Lösung mit Wasser verdünnt und mit Essigsäure neutralisiert. Die feinen roten Kristalle werden abfiltriert und aus einem Wasser-Ethanol-Gemisch unkristallisiert, wobei die Ausbeute 63% der Theorie (5,1 g) beträgt und der Schmelzpunkt bei 94 °C liegt.

$^1$H-NMR ($D_6$-DMSO): δ = 2,24 (s; 3H, -$CH_3$) 3,16-3,22 (m; 2H, -$C\underline{H_2}$-OH) 3,64-3,66 (m; 2H,NH-$C\underline{H_2}$) 3,86 (s; 3H, -$OCH_3$) 4,82 (br. s; 1H, OH, tauscht mit $D_2O$ aus) 4,93 (br; 1H, NH, tausch mit $D_2O$ aus) 7,04 (s; 1H, 6-H) 7,15 (s; 1H, 3-H)

MS (70 eV): m/e = 226 ($M^{+\cdot}$)

Stufe 4: 4-Amino-2-(2'-hydroxyethyl)amino-5-methoxy-toluol

3 g (15 mmol) 2-(2'-Hydroxyethyl)amino-4-nitro-5-methoxytoluol werden mit katalytischen Mengen Palladium/Kohlenstoff ( 5% Pd) in 100 ml absolutem Ethanol hydriert. Nach dem Abfiltrieren des Katalysators wird das Lösungsmittel vollständig abdestilliert und die grauen Kristalle aus Methanol umkristallisiert. Man erhält 2,2 g (83% der Theorie) 4-Amino-2-(2'-hydroxyethyl)amino-5-methoxy-toluol, das bei 148 °C schmilzt.

$^1$H-NMR ($D_6$-DMSO): δ = 2,01 (s; 3H, $CH_3$) 3,06 (m; 2H, -$C\underline{H_2}OH$) 3,61-3,66 (m; 2H, -$NHC\underline{H_2}$) 3,68 (s; 3H, $OCH_3$) 4,08 (br. s; 1H, OH tauscht mit $D_2O$ aus) 4,37 (br. s; 2H, $NH_2$ tauscht mit $D_2O$ aus) 4,76 (br. s; 1H, NH, tauscht mit $D_2O$ aus) 6,05 (s; 1H, 3-H) 6,55 (s; 1H, 6-H)

MS (70 ev): m/e 196 ($M^{+\cdot}$)

Beispiel B: Herstellung von 4-Amino-2-(2'-hydroxyethyl)amino-5-ethoxy-toluol

Stufe 1 und 2: 2-Chlorethyl-N-[(4-fluor-2-methyl-5-nitro)-phenyl]-carbamat

2-Chlorethyl-N-[4-fluor-2-methyl-5-nitro)-phenyl]-carbamat wird gemäß den im Beispiel A beschriebenen Stufen 1 und 2 aus 4-Fluor-2-methyl-anilin hergestellt.

Stufe 3: 2-(2'-Hydroxyethyl)amino-5-ethoxy-4-nitrotoluol

11 g (3,6 mmol) 2-Chlorethyl-N-[(4-fluor-2-methyl-5-nitro)-phenol]-carbamat werden in 10 ml Ethanol und 10 ml 10-prozentiger Natronlauge 1 Stunde unter Rückfluß erhitzt. Die Lösung wird auf Eis gegeben und mit Essigsäure neutralisiert. Der rote Niederschlag wird abfiltriert.Die Ausbeute beträgt 0,7 g (80% der Theorie) und der Schmelzpunkt 119 °C.

$^1$H-NMR ($D_6$-DMSO): δ = 1,31 (t, J = 7,0 Hz; 3H, $OCH_2C\underline{H_3}$) 2,17 (s; 3H, -$CH_3$) 3,10-3,16 (m; 2H-$C\underline{H_2}OH$) 3,58 (m; 2H, -NH-$C\underline{H_2}$) 4,04-4,08 (q, J = 6,9 Hz; 2H, -$OC\underline{H_2}CH_3$) 4,78 (br. s; 1H, NH) 4,90 (t, J = 5,6 Hz; 1H, OH) 6,96 (s; 1H, 6-H) 7,08 (s; 2H, 3-H)

MS (70 eV): m/e = 240 ($M^{+\cdot}$)

Stufe 4: 4-Amino-2-(2'-hydroxyethyl)amino-5-ethoxy-toluol

644 mg (2,7 mmol) 2-(2'-Hydroxyethyl)amino-5-ethoxy-4-nitro-toluol werden mit katalytischen Mengen Palladium/Kohlenstoff ( 5% Pd) in 50 ml Methanol hydriert. Nach dem Abfiltrieren des Katalysators wird das Lösungsmittel vollständig abdestilliert, wobei 428 mg (76% der Theorie) 4-Amino-2-(2'-hydroxyethyl)amino-5-ethoxytoluol dünnschichtchromatographisch rein anfallen. Die erhaltenen Kristalle schmelzen bei 128 °C.

$^1$H-NMR ($D_6$-DMSO): δ = 1,26 (t, 3H, $C\underline{H}_3$-$CH_2$-O-) 1,94 (s; 3H, -$CH_3$) 2,98 (m; 2H, -$CH_2$-OH) 3,57 (m; 2H, NH-$C\underline{H}_2$) 3,79-3,86 (q; 2H, $CH_3$-$C\underline{H}_2$-O-) 4,12 (br. s; 1H, OH, tauscht mit $D_2O$ aus) 4,69 (br. s; 1H, NH, tauscht mit $D_2O$ aus) 5,98 (s; 1H, 3-H) 6,48 (s; 1H, 6-H)

MS (70 eV): m/e = 210 ($M^{+\cdot}$)

Beispiel C: Herstellung von 4-Amino-2(2'-hydroxyethyl)amino-5-chlor-toluol-hydrochlorid

Stufe 1: 2-Amino-5-chlor-4-nitro-toluol

7 g (20 mmol) 2-Amino-5-chlor-toluol werden bei 10 °C in 10 ml $H_2SO_4$ gelöst. 2,05 ml rauchende $HNO_3$ werden in 10 ml konzentrierter $H_2SO_4$ gelöst und langsam zu der Lösung zugetropft. Die Lösung wird 3-4 Stunden bei Raumtemperatur gerührt und anschließend auf Eis gegeben. Die gelben Kristalle werden abgesaugt und aus Methanol umkristallisiert. Man erhält 6,4 g (70% der Theorie) 2-Amino-5-chlor-4-nitro-toluol, das bei 115 ° C schmilzt.

$^1$H-NMR ($D_6$-DMSO): δ = 2,13 (s; 3H, $CH_3$) 4,24 (br. s; 2H, $NH_2$) 7,26 (s; 2H, 3-H, 6-H)

MS (70 eV): m/e = 141 ($M^{+\cdot}$)

Stufe 2: 2-Chlorethyl-N-[(4-chlor-2-methyl-5-nitro)-phenyl]-carbamat

3 g (16 mmol) 4-Amino-5-chlor-toluol werden in 150 ml Dioxan mit 1,5 g Kalziumcarbonat auf 70 °C erhitzt. 3,6 ml (35 mmol) Chlorameisensäurechlorethylester werden zugetropft und die Lösung 3,5 Stunden bei 100 °C gerührt. Es wird filtriert, auf Eis gegeben und die hellen Kristalle abgesaugt. Es werden 4,5 g (94% der Theorie) 2-Chlorethyl-N-[(4-chlor-2-methyl-5-nitro)-phenyl]-carbamat erhalten, die bei 93 °C schmelzen.

$^1$H-NMR ($D_6$-DMSO): δ = 2,33 (s; 3H, -$CH_3$) 3,87 (t, J = 5,2 Hz; 2H, -$C\underline{H}_2$Cl) 4,39 (t, J = 5,2 Hz; 2H, -$C\underline{H}_2$-$CH_2$Cl) 7,59 (s; 1H, 6-H) 8,23 (s; 1H, 3-H) 9,51 (br. s; 1H, -NH)

MS (70 eV): m/e = 293 ($M^{+\cdot}$)

Stufe 3: 2-(2'-Hydroxyethyl)amino-4-nitro-5-chlor-toluol

2 g (6,8 mmol) 2-Chlorethyl-N-[(4-chlor-2-methyl-5-nitro)-phenyl]-carbamat werden in 10 ml Methanol auf 70 °C erhitzt. 8,2 ml (21 mmol) 10-prozentige Natronlauge werden zugetropft und 1,5 Stunden unter Rückfluß erhitzt. Die Lösung wird auf Eis gegeben und mit Essigsäure neutralisiert. Der entstandene Niederschlag wird abfiltriert und aus Ethanol/Wasser umkristallisiert. Man erhält 816 mg (52% der Theorie) orange Kristalle, die bei 110 °C schmelzen.

$^1$H-NMR ($D_6$-DMSO): δ = 2,15 (s; 3H, -$CH_3$) 3,22 (q, J = 5,7 Hz; 2H, -$C\underline{H}_2$OH) 3,58 (q, J = 5,8 Hz; 2H, -NH-$C\underline{H}_2$-)4,78 (t, J = 5,6 Hz, 1H, -OH, tauscht mit $D_2O$ aus) 5,53 (t, J = 5,3 Hz; 1H, -NH, tauscht mit $D_2O$ aus) 7,11 (s; 1H, 6-H) 7,29 (s; 1H, 3-H)

MS (70 eV): m/e = 230 ($M^{+\cdot}$)

Stufe 4: 4-Amino-2-(2'-hydroxyethyl)amino-5-chlor-toluol-hydrochlorid

670 mg (2,9 mmol) 2-(2'-Hydroxyethyl)amino-5-chlor-4-nitro-toluol und 625 g Zinn werden in 10 ml halbkonzentrierter Salzsäure 1 Stunde unter Rückfluß erhitzt. Nach dem Abkühlen wird mit 10 ml $H_2O$ verdünnt und die Mischung auf 40 ml 10-prozentige Natronlauge gegeben. Die ausgefallenen Zinn-Salze werden abfiltriert und das Filtrat 3mal mit jeweils 30 ml Methylenchlorid extrahiert. Nach dem Waschen mit Wasser wird über $Na_2SO_4$ getrocknet und das Lösungsmittel vollständig abdestilliert. Der ölige Rückstand wird in 20 ml Methanol ausgenommen und mit der äquimolaren Menge Salzsäure versetzt.

$^1$H-NMR ($D_6$-DMSO): δ = 1,93 (s; 3H, -$CH_3$) 3,05 (t, J = 6,0 Hz; 2H, -$CH_2$-OH) 3,33 (br.s, 1H, OH,

tauscht mit $D_2O$ aus) 3,59 (t,J = 5,9 Hz; 2H, NHCH₂) 4,72 (br.s; 2H, $NH_2$, tauscht mit $D_2O$ aus) 5,1 (br.s; 1H, NH, tauscht mit $D_2O$ aus) 6,75 (s; 1H, 3H) 6,95 (s; 1H, 6-H)

MS (70 eV): m/e = 200 ($M^{+\cdot}$)

Alle in der vorliegenden Anmeldung angegebenen Prozentzahlen stellen, sofern nicht anders vermerkt, Gewichtsprozent dar.

**Patentansprüche**

1. Mittel zur oxidativen Färbung von Haaren auf der Basis einer Entwicklersubstanz-Kupplersubstanz-Kombination sowie gegebenenfalls anderer Farbkomponenten und in Haarfärbemitteln üblichen Zusätzen, dadurch gekennzeichnet, daß es mindestens eine Kupplersubstanz der allgemeinen Formel

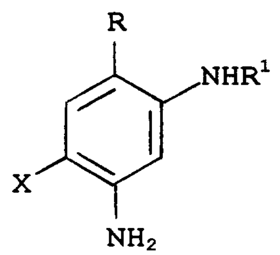

(I),

wobei R einen geradkettigen oder verzweigten $C_1$- bis $C_4$-Alkylrest bedeutet, X Chlor oder einen geradkettigen oder verzweigten $C_1$- bis $C_4$-Alkoxyrest und $R^1$ einen geradkettigen oder verzweigten $C_1$- bis $C_4$-Alkylrest oder einen geradkettigen oder verzweigten $C_2$- bis $C_4$-Hydroxyalkylrest darstellt, oder deren physiologisch verträgliches wasserlösliches Salz enthält.

2. Mittel nach Anspruch 1, dadurch gekennzeichnet, daß es die Kupplersubstanz der allgemeinen Formel (I) in einer Menge von 0,01 bis 5 Gewichtsprozent enthält.

3. Mittel nach Anspruch 1 oder 2, dadurch gekennzeichnet, daß es als Kupplersubstanz 4-Amino-2-(2'-hydroxyethyl)amino-5-ethoxy-toluol, 4-Amino-2-(2'-hydoxyethyl)amino-5-methoxy-toluol oder 4-Amino-2-(2'-hydroxyethyl)amino-5-chlor-toluol enthält.

4. Mittel nach einem der Ansprüche 1 bis 3, dadurch gekennzeichnet, daß es zusätzlich mindestens eine weitere Kupplersubstanz enthält, welche ausgewählt ist aus Resorcin, 4-Chlorresorcin, 4,6-Dichlorresorcin, 2-Methylresorcin, 2-Amino-4-(2'-hydroxyethylamino)anisol, m-Phenylendiamin, 5-Amino-2-methylphenol, 2,4-Diaminophenoxyethanol, 1-Naphthol,m-Aminophenol, 3-Amino-4-chlor-6-methylphenol, 3-Amino-2-methyl-phenol, 4-Amino-2-hydroxyphenoxyethanol, 4-Hydroxy-1,2-methylendioxybenzol ,4-(2'-Hydroxyethylamino)-1,2-methylendioxybenzol, 2,4-Diamino-5-ethoxytoluol, 4-Hydroxyindol, 4-Amino-5-fluor-2-hydroxy-toluol, 4-Amino-5-ethoxy-2-hydroxy-toluol und 3,5-Diamino-2,6-dimethoxypyridin.

5. Mittel nach einem der Ansprüche 1 bis 4, dadurch gekennzeichnet, daß die Entwicklersubstanz ausgewählt ist aus 1,4-Diaminobenzol, 2,5-Diamino-toluol, 2-(2'-Hydroxyethyl)-1,4-diaminobenzol, 4-Aminophenol, 4-Amino-2-aminomethyl-phenol, 4-Amino-3-methylphenol, 4-Amino-2-methoxymethylphenol, 4-Amino-2-ethoxymethylphenol,4,5-Diamino-1-methyl-pyrazol, 4,5-Diamino-1-isopropyl-pyrazol, 1-Benzyl-4,5-diaminopyrazol, 4,5-Diamino-1-methylbenzyl-pyrazol sowie Tetraaminopyrimidin oder deren physiologisch verträglichen Salzen.

6. Mittel nach einem der Ansprüche 1 bis 5, dadurch gekennzeichnet, daß die Gesamtmenge der Entwicklersubstanz-Kupplersubstanz-Kombination 0,1 bis 5,0 Gewichtsprozent beträgt.

7. Mittel nach einem der Ansprüche 1 bis 6, dadurch gekennzeichnet, daß es eine Farbkomponente enthält, welche ausgewählt ist aus 6-Amino-2-methylphenol, 2-Amino-5-methylphenol, C. I. Basic Violet 14 (C. I. 42 510), C. I. Basic Violet 2 (C. I. 42 520), 2-Amino-4,6-dinitrophenol, 2-Amino-5-(2'-hydroxyethylamino)-nitrobenzol, 4-(2'-Hydroxyethylamino)-3-nitrotoluol, 1-(2'-Ureidoethyl)amino-4-nitrobenzol, C. I. Acid Brown 4 (C. I. 14 805), 1,4-Diaminoanthrachinon und 1,4,5,8-Tetraaminoanthrachinon.

**8.** 2-Alkylamino-4-amino-1-alkylbenzol der allgemeinen Formel

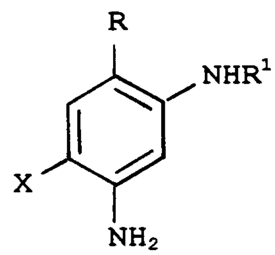

(I),

wobei R einen geradkettigen oder verzweigten $C_1$- bis $C_4$-Alkylrest bedeutet, X Chlor oder einen geradkettigen oder verzweigten $C_1$- bis $C_4$-Alkoxyrest und $R^1$ einen geradkettigen oder verzweigten $C_1$- bis $C_4$-Alkylrest oder einen geradkettigen oder verzweigten $C_2$- bis $C_4$-Hydroxyalkylrest darstellt.

**9.** 4-Amino-2-(2'-hydroxyethyl)amino-5-ethoxy-toluol.

**10.** 4-Amino-2-(2'-hydroxyethyl)amino-5-methoxy-toluol.

**11.** 4-Amino-2-(2'-hydroxyethyl)amino-5-chlor-toluol.

Europäisches Patentamt

# EUROPÄISCHER RECHERCHENBERICHT

Nummer der Anmeldung
EP 95 10 1867

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | DE-A-42 06 416 (WELLA AG) 2.September 1993<br>* das ganze Dokument *<br>--- | 1-11 | C07C217/84<br>C07C211/52<br>A61K7/13 |
| D,Y | DE-A-36 22 784 (WELLA AG) 21.Januar 1988<br>* das ganze Dokument *<br>--- | 1-11 | |
| D,Y | DE-A-40 28 661 (WELLA AG) 12.März 1992<br>* das ganze Dokument *<br>--- | 1-11 | |
| Y | DE-A-37 24 642 (WELLA AG) 2.Februar 1989<br>* das ganze Dokument *<br>--- | 1-11 | |
| Y | DE-C-38 06 237 (HANS SCHWARZKOPF GMBH)<br>12.Oktober 1989<br>* das ganze Dokument *<br>--- | 1-11 | |
| Y | BE-A-857 669 (L'OREAL) 10.Februar 1978<br>* das ganze Dokument *<br>----- | 1-11 | |

RECHERCHIERTE SACHGEBIETE (Int.Cl.6)
C07C
A61K

Der vorliegende Recherchenbericht wurde für alle Patentansprüche erstellt

| Recherchenort | Abschlußdatum der Recherche | Prüfer |
|---|---|---|
| MÜNCHEN | 24.Mai 1995 | Janus, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)